# EUROPEAN PATENT SPECIFICATION

(11) **EP 2 816 986 B1**
(45) Date of publication and mention of the grant of the patent: **28.03.2018**
(21) Application number: 13751566.4
(22) Date of filing: 19.02.2013
(51) Int. Cl.: A61F 9/008, A61F 9/009

(54) **LASER BEAM OPHTHALMOLOGICAL SURGERY APPARATUS**
LASERSTRAHL-AUGENCHIRURGIEVORRICHTUNG
APPAREIL DE CHIRURGIE OPHTALMIQUE PAR FAISCEAU LASER

(30) Priority: 24.02.2012 US 201213404127
(43) Date of publication of application: 31.12.2014
(73) Proprietor: Excelsius Medical Inc., Orlando, FL 32817 (US)
(72) Inventor: HUANG, Cheng-hao, Orlando, FL 32817 (US)
(74) Representative: Sloboshanin, Sergej
(86) International application number: PCT/US2013/026625
(87) International publication number: WO 2013/126316

(56) References cited:
- EP-A1- 1 731 120
- US-A- 4 583 539
- US-A- 5 505 723
- US-A- 5 549 632
- US-A- 5 599 340
- US-A1- 2006 129 141
- US-A1- 2006 217 688

## Description

### BACKGROUND OF THE INVENTION

This invention is related to refractive eye surgery and especially to refractive eye surgery using an articulated mirror-lens relay arm to project a random scanning of one or more laser beams in the ablation of cornea tissue to reshape the cornea of the eye.

The cornea is a thin shell with nearly concentric anterior and posterior surfaces and a central thickness of about 520 micrometers. It has an index of refractive of 1.377 and a nominal radius of curvature of 7.86 mm. The epithelium, forming the anterior surface of the cornea, is about 70 micrometers thick in young people at the center. Underlying the epithelium is a layer called Bowman's layer or Bowman's membrane, which is about 12 micrometers thick. This covers the anterior surface of the stroma, which makes up the bulk of the cornea and consists primarily of collagen fibers. The endothelium that forms the posterior layer of the cornea is a single layer of cells.

About three-quarter of the refractive power of the eye is determined by the curvature of the anterior surface of the cornea, so that changing the shape of the cornea offers a way to significantly reduce or eliminate refractive errors of the eye. The stroma is thick enough so that portions of its anterior region can be ablated away to change its profile and thus change the refractive power of the eye for corrective purposes, while leaving plenty of remaining stroma tissue.

Various lasers have been used for ophthalmic applications including the treatment of glaucoma, cataract and refractive surgery. For refractive surgeries (or corneal reshaping), ultraviolet (UV) lasers, such as excimer lasers at 193nm and fifth-harmonic Nd:YAG at 213nm have been used for large area surface corneal ablation in a process called photorefractive keratectomy (PRK) and for large area stroma ablation in a process called laser assisted in situ keratomileusis (LASIK). Corneal reshaping may also be performed by laser thermal coagulation currently conducted with Ho:YAG laser using a fiber-coupled contact and flying laser spots non-contact type process.

Refractive surgery has reached a new dimension due to the development of the excimer laser (193nm) and fifth harmonic solid state laser (190nm-215nm) being used to photo ablate the corneal tissue to reshape the cornea. In my prior U.S. Pat. No. 5,480,396 dated Jan. 2, 1996 for LASER BEAM OPHTHALMOLOGICAL SURGERY METHOD AND APPARATUS and U.S. Pat. No. 5,599,340 dated Feb. 4, 1997 for LASER BEAM OPHTHALMOLOGICAL SURGERY METHOD AND APPARATUS, single or plural beams are formed by splitting one laser beam, and uses a random scanning pattern of the beams to scan the laser beams over the cornea. Most of the latest refractive laser systems have used either one or two laser beams and a random scanning pattern.

However, all the refractive laser systems available in the market are similar in that they require the patient eye to align with the laser beam. In this case, the laser cabinet is bulky and one laser delivery arm is used to transfer the laser beam into a random scanning pattern on the cornea. The laser delivery arm is around one meter and the laser beam is turned down under the microscope in order to align the laser beam with the microscope's visual axis. The distance from the lower part of the laser delivery arm to the surface of the cornea is around 250 millimeters. The patient is lying down on a patient table that can be XYZ fine tuned in order to move the surface of the cornea until it reaches the focusing point of the microscope and laser delivery set up. In this kind of laser system organization, the patient is required to move constantly in order to align the patient eye to the laser beam. To prevent eye movement during the surgery, the surgeon uses a ring or other tool to stabilize the eye ball. Most of the modern laser systems employ an eye tracker system to track the eye movement but even a very complicated three dimensional eye tracking system cannot guide the laser beam to be normal incidence upon the corneal surface if the eye ball rotates.

U.S. Patent No. 5,599,340 has a UV laser with an XY scanned device located in a fixed delivery arm that delivers the laser beam to the cornea without any mechanical contact. The visual axis of a microscope is aligned with the UV laser beam since the microscope is used to monitor the ablation. A sophisticated movement patient table is used to move the patient's eye into alignment with the visual axis of the microscope and the UV laser beam. This requires significant effort for the surgeon to precisely align the beam with the eye. My U.S. Patent Application Serial No. 13/373,591 published as US2013131653 A1 uses a femtosecond laser where the laser beam pulses are sent through an XY-scanning device located in a main cabinet. The beam then travels through a mirror-lens relay optical arm to a hand piece, that contains an XYZ piezo stage and a very short focal length lens with a high numerical aperture, and is connected to the surface of the eye via a suction ring. This apparatus therefore aligns the laser beam with the eye to simplify the surgical procedure by removing the necessity and effort of the surgeon and patient of using a sophisticated movement patient table to align the eye with the laser beam. However, a millimeter sized laser spot and a 10 mm ablation area is required for UV laser refractive surgery so that a highly focused lens is not suitable for this application. In the UV laser application, the femtosecond laser's XYZ piezo stage is no longer required when using a long focal length scan, focusing lens with a low numerical aperture to cover the 10 mm ablation area.

The present invention proposes a design for a laser system in which the laser beam aligns with the patient eye rather than having the patient's eye aligned to the UV laser beam. The laser system uses a mirror-lens relay optical arm to transfer a random scanning pattern after XY galvanometer to a remote position that is around 1.5 meters away from the scanner device and with the same characteristics of the scanned laser beams. The laser beam then passes into the scan, focusing lens of the hand piece and is directed to the patient's eye.

In greater detail, the hand piece consists of two sections: the scan lens section and the viewing/centering section. The scan-lens section, attached to the far end of the mirror-lens relay optical arm, contains a high F-number (low NA) F-Theta scan, focusing lens with a focusing length of around 100mm that allows the focusing point of the scanned laser beam to pass into the viewing/centering section of the hand piece. The scan lens also converts a divergent beam into a parallel beam which has an advantage on the ablation efficiency curve on the corneal surface. The viewing section of the hand piece includes a turn down mirror which has a working distance of approximately 50mm from the lower surface of the hand piece to the eye surface and is arranged in such a way that it can be in contact with and stabilize the eye by using the eye stabilization and distance control unit. A power detector is attached to the viewing section to monitor the power level of the laser beam. The viewing section also contains four centering lights used to aid in properly centering the laser beam onto the cornea. Suction for debris removal is located in the eye stabilization and distance control unit to remove the laser ablation tissue particles through tiny ventilation holes. In this laser delivery configuration, the laser beam is directed by the surgeon to the eye so there is no need to move the patient during the surgery. Since the hand piece with eye stabilization and distance control unit is placed by the surgeon on the top of the eye to align the laser beam with the eye, there is no need to use an eye tracker to detect eye movement. In the new system, even if the eye is moving during the surgery, the laser beam remains normal incidence on the eye, which the eye tracker system cannot accomplish in existing systems.

### SUMMARY OF THE INVENTION

This invention is related to a laser ophthalmological surgery apparatus as set forth in independent claim 1. Described is also a method of ablating eye tissue includes generating a UV laser beam, such as with an excimer laser, and directing the generated laser beam through a manually activated shutter and through a homogenizer and onto a galvanometer xy-scanner. The scanner generates an overlapping random pattern laser beam signal. A selected hand piece has a f-theta telecentric scan focusing lens therein for converting the laser beam from a divergent to a parallel scanning beam and an eye stabilization and distance control member extending therefrom, which eye stabilization and distance control member has an open center area and an eye contact edge thereon. The selected hand piece also has at least one aligning light thereon for aligning the eye stabilization and distance control member with a patient's eye and may have a video camera attached thereto. The generated laser beam random pattern is directed through an articulated mirror-lens relay arm to the hand piece scan lens mounted in the hand piece and through the open center of the eye stabilization and distance control member. The hand piece is manipulated on the end of the articulated mirror-lens relay arm to align the eye stabilization and distance control unit on a patient's eye and then is further manipulated to bring the eye stabilization and distance control member eye contact edge into eye contact with a patient's eye. The random pattern laser beam from the laser scanner is then impinged onto the surface of the patient's eye responsive to activation of the shutter to ablate tissue from the surface of the patient's eye. Thus, surgical ablation is performed on a patient's eye with a random overlapping laser beam through a hand manipulated hand piece in contact with the patient's eye.

A laser ophthalmological surgery apparatus according to the present invention has a UV laser, such as an excimer laser, for generating a UV laser beam which beam is applied to a control shutter and through a homogenizer and into a galvanometer xy-scanner. The scanner produces a predetermined random overlapping scanning pattern. A computer generates the random scanning pattern signal for controlling the scanner to produce the random overlapping scanning pattern from the input laser beam. A hand piece has a f-theta telecentric scan focusing lens therein for converting the laser beam from a divergent to a parallel scanning beam and an eye stabilization and distance control member attached to and extending therefrom. The eye stabilization and distance control member has an open center area and an eye contact edge. One or more, but typically four, alignment lights are mounted on the hand piece for aligning the eye stabilization and distance control member on a patient's eye.

An articulated mirror-lens relay arm has a hand piece attached to one end thereof and the other end positioned for receiving the scanned laser beam and directing the laser beam to the hand piece and through an open center area of the eye stabilization and distance control member. The hand piece can be manipulated to position it to direct the laser beam onto a patient's eye by manipulating the articulated mirror-lens relay arm to position the eye stabilization and distance control member eye contact edge into contact with a patient's eye to stabilize a patient's eye for ablating tissue therefrom in a random overlapping pattern with the laser beam. A vacuum pump has a vacuum line connected to the hand piece to remove the eye's ablated tissue. Surgical ablation can thus be performed on a patient's eye with a random overlapping laser beam through a hand manipulated hand piece in contact with the patient's eye.

### BRIEF DESCRIPTION OF THE DRAWINGS

The accompanying drawings, which are included to provide further understanding of the invention and are incorporated in and constitute a part of the specification, illustrate an embodiment of the invention and together with the description, serve to explain the principles of the invention.

### In the drawings:

FIG. 1 is a block diagram of an ophthalmologic laser surgery apparatus in accordance with the present invention;
FIG. 2 is a side diagrammatic view of the hand piece of Figure 1 positioned on a patient's eye;
FIG. 3 is a top diagrammatic view of the hand piece of Figure 2; and
FIG. 4 shows a randomized scanning pattern where the numbers are in the order in which the first nine laser beam spots are scanned. While the laser spots overlap, the spots shown in this figure are non-overlapping for the sake of clarity.

### DETAILED DESCRIPTION OF EXEMPLARY EMBODIMENT

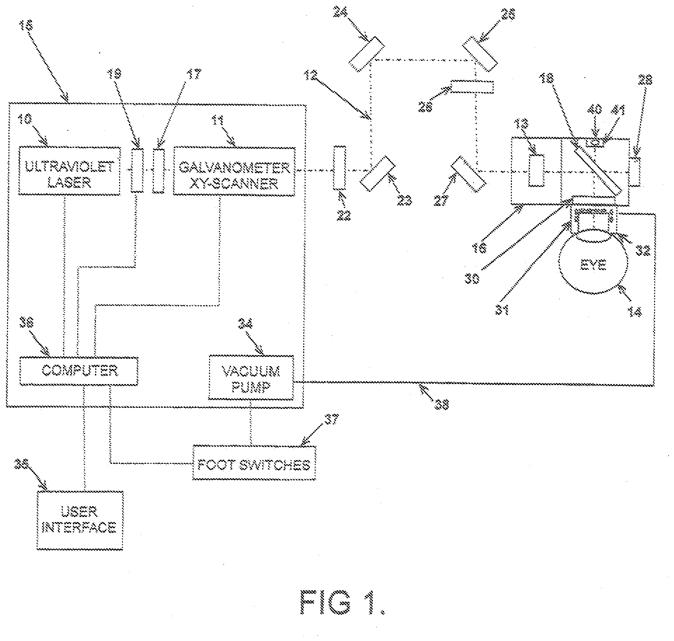

A method of ablating eye tissue, as illustrated in Figures 1 - 3, generates a pulse laser beam from an excimer or ultra violet (UV) laser 10 and applies the generated laser beam to a laser scanner 11 to generate an overlapping random scanning pattern of laser pulses. The overlapping random scanning pattern is remotely relayed by an articulated optical arm 12 to a scan, focusing lens 13 which focuses the laser beam spot and overlapping random scanning pattern onto a patient's eye 14 to ablate a predetermined pattern of eye tissue in the patient's eye. The patient's eye 14 is thus ablated in a predetermined overlapping random scanning pattern with a controlled focused laser beam of an excimer or UV laser 10.

The ophthalmological apparatus has a main cabinet 15 and a hand piece 16 connected thereto with the articulated mirror-lens relay arm 12. The excimer laser 10 is positioned in the main cabinet 15 and has a laser beam output of laser pulses. A laser beam homogenizer 17 is positioned to homogenize the laser beam to smooth out irregularities in the laser beam before scanning the laser beam into an overlapping random scanning pattern of laser pulses. The scan lens 13 is located in the hand piece 16 to receive the overlapping random scanning pattern of laser pulses from the laser scanner 11 which are centered on the center axis of the focusing lens 13. The scan, focusing lens 13 is positioned to focus the predetermined overlapping random scanning pattern of laser pulses onto a turn down lens 18 and onto a patient's eye 14 to ablate a predetermined sub-area of eye tissue. Thus, a patient's eye has a predetermined area thereon ablated by overlapping random scanning pattern of laser pulses.

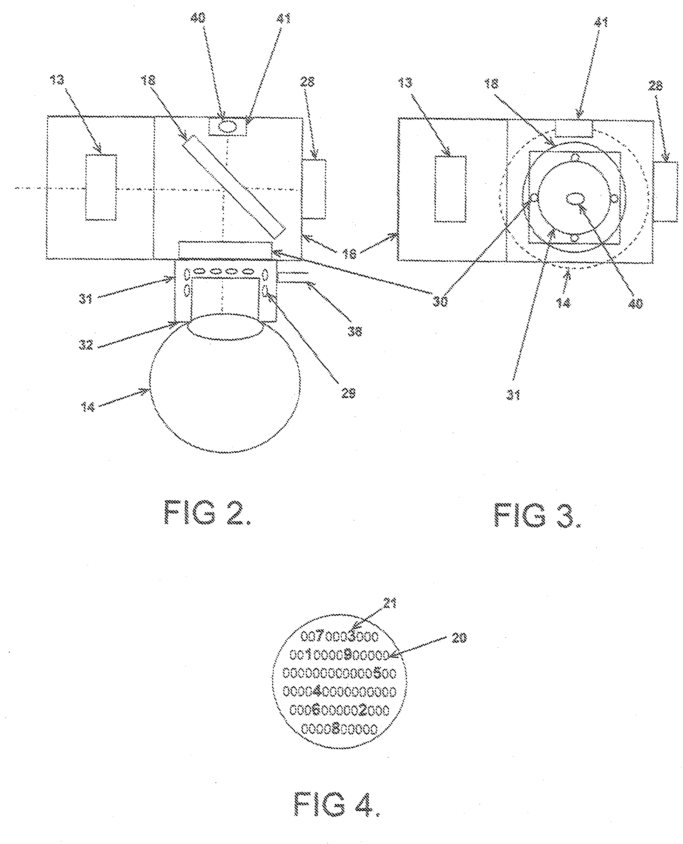

The laser ophthalmological surgery apparatus in accordance with the present invention as seen in the drawings includes a user interface connected to a main cabinet 15 in FIG. 1. Within the main cabinet, laser pulses are generated with UV laser 10 that are deflected by XY-galvanometers (galvos) 17 to create an overlapping random scanning pattern 20, such as shown in Fig. 4. The overlapping random scanning pattern 20 of the laser beam spots 21 passes through relay lens 22 and 26 and through the articulated arm 12 having mirrors 23, 24, 25 and 27 and into the remote hand piece 16 where it has same characteristics and properties as the output from the scanning galvos 11. Inside the hand piece 16, the scan, focusing lens 13 is used to reduce the size of the laser beam spots and the size of the overlapping random scanning pattern 20 according to a predetermined pattern on the cornea. A power detector or meter 28 is used to monitor the power level of the laser beam. Four centering lights 30 are used to position the hand piece 16 eye stabilization and distance control unit on the eye 14. The hand piece 16 is then positioned in contact with the eye 14 via the eye stabilization and distance control unit 31 eye contact surface 32 which levels the focused laser beam spot onto the eye and stabilizes the eye 14. A tissue debris suction tube 38 is attached on the eye stabilization and distance control unit 31 to remove the laser ablation particles through suction vents 29 to avoid the interference of tissue particles and is connected to a vacuum pump 34. A user interface 35 is connected to a computer 36 which in turn is used to control the vacuum pump 34 and the shutter 19 as well as the galvanometer xy-scanner 11. The shutter 19 opens and closes to control the laser pulses that are applied through the homogenizer 17 and to the scanner 11.

Following the light path in greater detail, the light pulse generator is an excimer laser 10 (wavelength 193nm) or fifth harmonic generated solid state laser (wavelength is from 193nm to 213nm) with a pulse width less than 20 nanosecond and a pulse repetition rate from 100Hz to several thousand Hz. The laser beam is blocked by the shutter 19 until the foot switch 37 is depressed thereby activating a switch. While the foot switch 37 is pressed, the laser beam is allowed to continue to the homogenizer 17 where the laser beam spot size is homogenized, i.e. smooths out the irregularities in the laser beam. The beam continues to the XY-galvanometer 11 where it is deflected to create an overlapping random scanning pattern 20, as shown in FIG. 4 (which figure shows non-overlapping spots for clarity). The overlapping random scanning pattern 20 passes through relay lens 22 and through the articulated mirror arm 12 having mirrors 23 to 25 therein and into the relay lens 26 and onto mirror 27. The purpose of the lenses and mirrors is so that the overlapping random scanning pattern 20 enters the hand piece 16 with the same characteristics and properties as the output scanned from the galvos 11.

Inside the hand piece 16, a compact scan, focusing lens 13 is used to reduce the spot size of the laser beam spots to less than 1mm and the size of the overlapping random scanning pattern 20 to a diameter of less than 10mm. The turn-down mirror 18 directs the laser beam towards the eye while a power meter 28 detects the laser beam's power.

The hand piece 16 is attached to the eye 14 with a disposable eye stabilization and distance control unit 31 with the eye contact edge 32 contacting the eye. The ablated tissue particles are removed through suction vents 29 by the vacuum pump 34 having a line in the eye stabilization and distance control unit 31 via a debris suction tube 38 that is attached to the eye stabilization and distance control unit vacuum vents 29. The eye stabilization and distance control unit 31 is placed on the eye 14 to correctly level the laser beam focusing point and to stabilize eye movement. The eye itself has some minor movement during the surgery, however, the laser beam is always maintained at a normal incidence to the eye since the hand piece has the same movement as the eye movement. Centering lights 30 are used to delineate the target area. A small cam mirror 40 directs the view of the eye with the centering lights to a video camera 41 so that the system's computer 13 can receive the video signal to detect the pupil location to provide feedback during the centering procedure.

The ablation patterns generated by this laser apparatus include but are not limited to predetermined myopia, hyperopia and astigmatism patterns. The computer randomly rearranges the ordered ablation data into a new random sequence of ablation. The laser beam spots are delivered to cornea in overlapping random patterns.

The process for refractive eye surgery uses the apparatus described having an articulated mirror-lens relay arm to project a random scanning of one or more UV laser beams to ablate cornea tissue to reshape the cornea of the eye. The method includes ablating eye tissue by generating a UV laser beam, such as with an excimer laser 10, and directing the generated laser beam through a manually activated shutter 19 and through a homogenizer 17 and onto a galvanometer xy-scanner 11. The scanner generates an overlapping random pattern laser beam signal. A selected hand piece 16 has a focusing lens 13 therein and an eye stabilization and distance control member 31 extending therefrom, which eye stabilization and distance control member has an open center area and an eye contact edge 32 thereon. The selected hand piece 16 also has at least one aligning light 30 thereon for aligning the eye stabilization and distance control member 31 with a patient's eye and may have a video camera 41 attached thereto. The generated laser beam random pattern is directed through an articulated mirror-lens relay arm 12 to the hand piece scan lens 13 mounted in the hand piece and through the open center of the eye stabilization and distance control member 31. The hand piece 16 is manipulated on the end of the articulated mirror-lens relay arm 12 to align the eye stabilization and distance control unit 31 on a patient's eye 14 and then is further manipulated to bring the eye stabilization and distance control member eye contact edge 32 into eye contact with a patient's eye 14. The random pattern laser beam from the laser scanner 11 is then impinged onto the surface of the patient's eye responsive to activation of the shutter 19 to ablate tissue from the surface of the patient's eye. Thus, surgical ablation is performed on a patient's eye 14 with a random overlapping laser beam through a hand manipulated hand piece 16 in contact with the patient's eye.

It should be clear at this time that a process and an apparatus for performing refractive eye surgery by surgical ablation on a patient's eye has been shown which uses a random overlapping laser beam through a hand manipulated hand piece in contact with the patient's eye. However the present invention is not to be considered limited to the forms shown which is to be considered illustrative rather than restrictive.

## Claims

1. A laser ophthalmological surgery apparatus having
a UV laser (10) for generating a UV laser beam;
a scanner (11) positioned for receiving said laser beam from said laser and producing a predetermined scanning pattern output therefrom; and
a computer (36) for generating a random scanning pattern signal, said computer (36) being connected to said scanner (11) for controlling said scanner to produce a random overlapping scanning pattern from said laser beam, **characterized by**:
a hand piece (16) having an f-theta scan focusing lens (13) therein for converting the laser beam from a divergent to a parallel scanning beam, said hand piece having
an eye stabilization and distance control member (31) attached to and extending from said hand piece (16), said eye stabilization and distance control member (31) having an open center area and an eye contact edge (32);
at least one alignment light (30) for aligning said eye stabilization and distance control member (31) on a patient's eye (14); and
an articulated mirror-lens relay arm (12) having said hand piece (16) attached to one end thereof and the other end positioned for receiving said scanned laser beam and directing said scanned laser beam through said f-theta scan focusing lens in said hand piece (16) and through said open center area of said eye stabilization and distance control member (31) whereby said articulated mirror-lens relay arm (12) is maneuverable to allow said hand piece (16) on the end thereof to position said eye stabilization and distance control member (31) into contact with said eye (14) to stabilize a patient's eye for ablating tissue with the parallel scanning laser beam; and
whereby surgical ablation can be performed on a patient's eye (14) with a random overlapping laser beam through the hand manipulated hand piece (16) in contact with the patient's eye (14).

2. The laser ophthalmological surgery apparatus in accordance with claim 1 including a video camera (41) mounted on said hand piece (16) for recording the open center area of the eye stabilization and distance control member (31).

3. The laser ophthalmological surgery apparatus in accordance with claim 2 in which said hand piece (16) has multiple alignment lights (30) thereon.

4. The laser ophthalmological surgery apparatus in accordance with claim 3 including a vacuum pump (34) having a suction line (38) connected therefrom to said hand piece (16) to remove ablated eye tissue while surgically ablating a patient's eye (14).

5. The laser ophthalmological surgery apparatus in accordance with claim 4 including a beam homogenizer (17) for smoothing out irregularities in the laser beam.

6. The laser ophthalmological surgery apparatus in accordance with claim 5 including a shutter (19) located between the UV laser (10) and the scanner (11).

## Patentansprüche

1. Laservorrichtung für ophthalmologische Chirurgie umfassend:
einen UV-Laser (10) zum Erzeugen eines UV-Laserstrahls;
einen Scanner (11), der für das Empfangen des Laserstrahls von dem Laser positioniert ist und daraus eine vorherbestimmte Scan-Muster-Ausgabe herstellt; und
einen Computer (36) zum Erzeugen eines zufälligen Scanmustersignals, wobei der Computer (36) mit dem Scanner (11) zum Steuern des Scanners verbunden ist, um ein zufälliges, sich überlappendes Scanmuster aus dem Laserstrahl herzustellen, **gekennzeichnet durch:**
ein Handstück (16), das in sich eine F-Theta-Scan-Fokussierungslinse (13) zum Konvertieren des Laserstrahls von einem divergierenden zu einem parallel scannenden Laserstrahl aufweist, wobei das Handstück
ein Augenstabilisierungs- und Abstandssteuerungselement (31) aufweist, das an dem Handstück (16) befestigt ist und sich von ihm weg erstreckt, wobei das Augenstabilisierungs- und Abstandssteuerungselement (31) einen offenen Mittebereich und einen Augenkontaktrand (32) aufweist;
wenigstens ein Ausrichtungslicht (30) zum Ausrichten des Augenstabilisierungs- und Abstandssteuerungselements (31) auf einem Patientenauge (14);
und einen angelenkten Spiegellinsen-Übertragungsarm (12), an dessen einem Ende das Handstück (16) befestigt ist und dessen anderes Ende für die Aufnahme des gescannten Laserstrahls und das Richten des gescannten Laserstrahls durch die F-Theta-Scan-Fokussierungslinse in dem Handstück (16) und durch den offenen Mittenbereich des Augenstabilisierungs- und Abstandssteuerungselements (31) positioniert ist, wobei der angelenkte Spiegellinsen-Übertragungsarm (12) manövrierbar ist, um es dem Handstück (16) auf seinem Ende zu erlauben, das Augenstabilisierungs- und Abstandssteuerungselement (31) in Kontakt mit dem Auge (14) zu positionieren, um ein Patientenauge zu stabilisieren, um Gewebe mit dem parallel scannenden Laserstrahl abzutragen; und
wobei die chirurgische Abtragung an einem Patientenauge (14) mit einem sich zufällig überlappenden Laserstrahl durch das handbediente Handstück (16) in Kontakt mit dem Patientenauge (14) durchgeführt werden kann.

2. Laservorrichtung für ophthalmologische Chirurgie nach Anspruch 1 umfassend eine Videokamera (41), die auf dem Handstück (16) angebracht ist, um den offenen Mittenbereich des Augenstabilisierungs- und Abstandssteuerungselements (31) aufzunehmen.

3. Laservorrichtung für ophthalmologische Chirurgie nach Anspruch 2, bei der auf dem Handstück (16) mehrfache Ausrichtungslichter (30) angebracht sind.

4. Laservorrichtung für ophthalmologische Chirurgie nach Anspruch 3 umfassend eine Vakuumpumpe (34), die eine Ansaugleitung (38) aufweist, die von dieser zu dem Handstück (16) verbunden ist, um abgetragenes Augengewebe zu entfernen, während ein Patientenauge (14) chirurgisch abgetragen wird.

5. Laservorrichtung für ophthalmologische Chirurgie nach Anspruch 4 umfassend einen Strahlhomogenisierer (17) zum Glätten von Unregelmäßigkeiten in dem Laserstrahl.

6. Laservorrichtung für ophthalmologische Chirurgie nach Anspruch 5 umfassend einen Verschluss (19), der zwischen dem UV-Laser (10) und dem Scanner (11) angeordnet ist.

## Revendications

1. Appareil de chirurgie ophtalmologique laser ayant
un laser UV (10) pour engendrer un faisceau laser UV ;
un scanner (11) positionné pour recevoir ledit faisceau laser issu dudit laser et produire une sortie de motif de balayage prédéterminée à partir de celui-ci ; et
un ordinateur (36) pour engendrer un signal de motif de balayage aléatoire, ledit ordinateur (36) étant raccordé audit scanner (11) pour commander audit scanner de produire un motif de balayage chevauchant aléatoire à partir dudit faisceau laser,
**caractérisé par** :
une pièce à main (16) ayant une lentille de focalisation de balayage f-thêta (13) en son sein pour convertir le faisceau laser d'un faisceau de balayage divergent en un faisceau de balayage parallèle, ladite pièce à main ayant
un organe de stabilisation de l'oeil et de commande de distance à l'oeil (31) fixé sur et s'étendant à partir de ladite pièce à main (16), ledit organe de stabilisation de l'oeil et de commande de distance à l'oeil (31) ayant une zone centrale ouverte et un bord de contact oculaire (32) ;
au moins une lumière d'alignement (30) pour aligner ledit organe de stabilisation de l'oeil et de commande de distance à l'oeil (31) sur l'oeil d'un patient (14) ; et
un bras de relais miroir-lentille articulé (12) ayant ladite pièce à main (16) fixée sur une extrémité de celui-ci et l'autre extrémité positionnée pour recevoir ledit faisceau laser balayé et diriger ledit faisceau laser balayé à travers ladite lentille de focalisation de balayage f-thêta dans ladite pièce à main (16) et à travers ladite zone centrale ouverte dudit organe de stabilisation de l'oeil et de commande de distance à l'oeil (31), par quel moyen ledit bras de relais miroir-lentille articulé (12) est manoeuvrable afin de permettre à ladite pièce à main (16) sur l'extrémité de celui-ci de positionner ledit organe de stabilisation de l'oeil et de commande de distance à l'oeil (31) en contact avec ledit oeil (14) afin de stabiliser l'oeil d'un patient pour réaliser une ablation de tissu avec le faisceau laser de balayage parallèle ; et
par quel moyen une ablation chirurgicale peut être effectuée sur l'oeil d'un patient (14) avec un faisceau laser chevauchant aléatoire par l'intermédiaire de la pièce à main (16) manipulée à la main en contact avec l'oeil du patient (14).

2. Appareil de chirurgie ophtalmologique laser conformément à la revendication 1 incluant une caméra vidéo (41) montée sur ladite pièce à main (16) pour enregistrer la zone centrale ouverte de l'organe de stabilisation de l'oeil et de commande de distance à l'oeil (31).

3. Appareil de chirurgie ophtalmologique laser conformément à la revendication 2 dans lequel ladite pièce à main (16) présente de multiples lumières d'alignement (30) sur celle-ci.

4. Appareil de chirurgie ophtalmologique laser conformément à la revendication 3 incluant une pompe à vide (34) ayant une conduite d'aspiration (38) raccordée à partir de celle-ci à ladite pièce à main (16) afin de retirer du tissu oculaire ayant subi une ablation tout en réalisant une ablation chirurgicale de l'oeil d'un patient (14).

5. Appareil de chirurgie ophtalmologique laser conformément à la revendication 4 incluant un homogénéisateur de faisceau (17) pour lisser des irrégularités dans le faisceau laser.

6. Appareil de chirurgie ophtalmologique laser conformément à la revendication 5 incluant un obturateur (19) situé entre le laser UV (10) et le scanner (11).
